(19) Europäisches Patentamt
European Patent Office
Office européen des brevets

(11) **EP 4 528 648 A1**

(12) **EUROPEAN PATENT APPLICATION**

(43) Date of publication:
**26.03.2025  Bulletin 2025/13**

(51) International Patent Classification (IPC):
*G06T 11/00* *(2006.01)*

(21) Application number: **24156011.9**

(52) Cooperative Patent Classification (CPC):
**G06T 11/005;** G06T 2211/408; G06T 2211/452

(22) Date of filing: **06.02.2024**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Siemens Healthineers AG
91301 Forchheim (DE)**

(72) Inventors:
- **Erath, Julien
  91054 Erlangen (DE)**
- **Petersilka, Martin
  91325 Adelsdorf (DE)**
- **Stierstorfer, Karl
  91052 Erlangen (DE)**
- **Sunnegaardh, Johan
  91325 Adelsdorf (DE)**

(74) Representative: **Siemens Healthineers
Patent Attorneys
Postfach 22 16 34
80506 München (DE)**

(54) **REDUCTION OF ARTIFACTS IN SPECTRAL COMPUTED TOMOGRAPHY IMAGE DATA**

(57)     Method for the reduction of artifacts in spectral computed tomography image data, the method comprising:

(a) receiving computed tomography measurement data with sets of measurement values, wherein one set corresponds to one detector element readout of the measurement data comprising measurement values from at least two different x-ray spectra;

(b) determining an error estimate per set of measurement values;

(c) defining one replacement reference value per set of measurement values;

(d) applying a function that maps the measurement values to corrected values, the corrected values being in between the mapped measurement value and the replacement reference value of the respective set, such that for larger errors the corrected value is closer to or at the replacement reference value and for smaller errors the corrected value is closer to or at the measurement value;

(e) reconstructing computed tomography image data based on the corrected values.

FIG 5

$200$

$(S_1, S_2, ..., S_N)$ — $201$

$W$ — $202$

$S^*$ — $203$

$S_k \longrightarrow S_k^{corr}$ — $204$

$205$

**Description**

The invention relates to a method for the reduction of artifacts in spectral computed tomography image data, a method for providing spectral computed tomography image data, a computer program, a computer-readable storage medium, and a computed tomography system.

Independent of the grammatical term usage, individuals with male, female or other gender identities are included within the term.

In spectral computed tomography (CT), x-ray attenuation measurements from multiple x-ray spectra, e.g. from two different x-ray spectra, are acquired from an object of interest. For example, to generate the measurements with different x-ray spectra, multiple measurements with different x-ray source voltages or beam filtrations may be used. Additionally or alternative, energy-resolved detectors, such as photon counting or dual layer detectors, may be deployed. The measured x-ray attenuation depends on the material that is scanned and the applied x-ray spectrum. Therefore, differences between attenuation measurements acquired for different x-ray spectra may be used to differentiate between materials that have the same x-ray attenuation for a single x-ray spectrum.

The mathematical problem of decomposing measured x-ray attenuations for different spectra into a basis suitable for clinical applications, e.g., water and iodine, is called material decomposition. Errors that occur prior to material decomposition may be caused by physical effects such as scattered radiation, extra-focal radiation, or very low signal counts in low dose situations. Errors may also be caused by imperfect data correction steps intended to correct for physical effects occurring during data acquisition. Typically, material decomposition does not reduce these errors. On the contrary, material decomposition tends to even increase the errors. Hence, any errors in the data or images prior to material decomposition get amplified and may result in image artifacts. Image artifacts may, for example, comprise streak artifacts, cupping artifacts and degraded contrast-to-noise ratio of the images.

In state-of-the-art CT systems, to minimize data errors prior to material decomposition, typically much effort is put in calibration and computational steps to correct for physical effects as accurately as possible.

Furthermore, the data acquisition modes available for spectral acquisition may be restricted in order to reduce the physical effects causing image artifacts. For example, the x-ray beam collimation may be reduced in order to reduce scattered radiation. However, this can have a direct negative implication on the clinical application: a smaller collimation typically results in a longer examination time and higher risk of motion artifacts. A longer examination time may also increase the amount of contrast agent required for the examination.

It is therefore an object of the invention to provide a means, in particular a method, to reduce the occurrence of image artifacts.

This object is met or exceeded by a method according to claim 1, a method according to claim 12, a computer program according to claim 13, a computer-readable storage medium according to claim 14, and a computed tomography system according to claim 15. Further advantages and features result from the dependent claims, the description and the attached figures.

According to a first aspect of the invention, a Method for the reduction of artifacts in spectral computed tomography image data is provided. The method comprises:

(a) receiving computed tomography measurement data with sets of measurement values, wherein one set corresponds to one detector element readout of the measurement data comprising measurement values from at least two different x-ray spectra;
(b) determining and/or receiving an error estimate per set of measurement values;
(c) defining one replacement reference value per set of measurement values for at least some of the sets;
(d) applying a function that maps the measurement values to corrected values, the corrected values being in between the mapped measurement value and the replacement reference value of the respective set, the distance of the corrected value to each depending on the error estimate such that for larger errors the corrected value is closer to or at the replacement reference value and for smaller errors the corrected value is closer to or at the measurement value;
(e) reconstructing computed tomography image data based on the corrected values.

Advantageously, the method may allow to reduce artifacts, in particular due to the step for mapping measurement values to the corrected values. For example, artifacts comprising streak artifacts may thus be reduced. Thus, for example, more challenging modes, such as dual source acquisition or large collimation may be enabled without introducing prohibitively strong artifacts. The method may be particularly advantageous, if data errors occur in relatively small portions of the whole measured data. Hence, preferably, only some data, in particular a minority of data, comprise substantial errors. For example, the method may be particularly advantageous, if less than 10% of the measured data have significant data errors. Small portions of data may be so erroneous that, without a correction, an attempt to perform a material decomposition may likely only add artifacts to the image. It has been found, that even only very few measurement values that are affected by errors, may have a large influence on artifacts on the image over a large part of the image. In particular even far less than 10% of the data may be affected by errors, but still lead to significant artifacts on the image. For example, only measurement data associated with particular x-rays from a particular direction may be affected. Advantageously, this effect of few erroneous measurement values having a negative impact on the whole image may advantageously be avoided by this method. On the other hand, these very few data, due to their relatively low number, may be of relatively minor importance for the spectral decomposition itself, such that still sufficient spectral information may remain.

The term "computed tomography (CT) measurement data" may be understood broadly in the context of this invention. It generally describes data acquired with a computed tomography system comprising measurement values. In the context of this invention, the x-ray intensity measurement data may in particular be x-ray intensity measurement data and/or attenuation measurement data. For example, attenuation measurement data may be derived from x-ray intensity

$$\mu = -\log{}^I\!/\!_{I_0}$$

measurement by applying a negative algorithm, such $\qquad$ with $\mu$ being the attenuation, $I$ being the x-ray intensity and $I_0$ being the x-ray intensity that would be measured without an attenuating object. The method according to the invention is generally applicable to both, x-ray intensity measurement data and attenuation measurement data. The measurement values may be acquired by sending x-rays from an x-ray source, in particular an x-ray tube, at least one x-ray detector. For example, the at least one x-ray detector may comprise a row and/or an array of x-ray detectors. Typically, an object is placed between the x-ray source and the at least one x-ray detector, such that the x-rays are attenuated depending on the material, e.g. tissue material, of the object before reaching the detector. For example, the object may be part of a patient. Typically, the x-rays generated by the x-ray source comprise a spectrum of x-ray frequencies. Depending on the specific spectrum of x-rays and the material that is in the pathway of the x-rays, the attenuation of the x-rays may differ. Recording the x-ray attenuation for different spectra may thus help to distinguish between different materials.

In the context of this invention, the measurement values may be put together in sets of measurement values. One set corresponds to one detector element readout of the measurement data comprising measurement values from at least two different x-ray spectra. The term "detector element readout" is to be understood broadly in the context of this invention. It generally describes a part of the at least one x-ray detector of the CT system. A detector element may correspond to a group of detector pixels or, preferably, to a single detector pixel. The detector element readout may, in particular, be a detector pixel readout. For example, the at least one x-ray detector may be an energy resolved detector, such as a photon counting detector or a dual layer detector. At least one energy resolved detector may be used to detect different x-ray spectra. Additionally or alternatively, for example, different x-ray spectra may be generated by applying different x-ray source voltages and/or different beam filtrations. Due to the sets comprising measurement values from at least two different x-ray spectra, a reconstruction of spectral data, allowing a better distinction between some materials may be enabled. For example, receiving the computed tomography measurement data may comprise retrieving the data from a database and/or directly receiving the data during a CT scan.

For the sets of measurement values an error estimate is determined and/or received. In particular one error estimate per set of measurement values may be determined and/or received. Hence, every detector element readout may be associated with an error. For example, the error estimate may be determined and/or chosen such that the error estimate of one of the measurement values of a set is chosen that yields the highest error estimate out of the error estimates for the measurement values of one set. For example, the error estimate may be stored in a database, such as in a database together with the measurement data. In this case, the error may be retrieved together with the measurement data. For example, the error estimate may be estimated by an external processor and provided for the method. Alternatively, the error estimate may be determined during this method itself. The error estimate may be an error estimate on an error scale that defines the severity of the error. The error estimate may be an estimation that defines whether there is an error or whether there is no error. The error estimate may be defined according to an error metric. A substantial error may be defined to exist, if the error metric is above a defined threshold.

For at least some of the sets of measurement values, a replacement reference value is defined. The replacement reference value may be defined based on the values of the measurement values of the set. Optionally, for some of the sets, in particular sets that have an error estimate that is below a threshold or that are determined not to have a substantial error, the defining of a replacement reference value may be omitted. Accordingly, the following step of applying a function may, optionally, only be applied to sets for which a replacement reference value has been defined. The replacement reference value may serve as a reference for the mapping of the measurement values to the corrected values.

The function that maps the measurement values to corrected values is in particular applied based on the error estimate and the replacement reference value. The output of the function is in particular a respective corrected value. The corrected values are in between the mapped measurement values and the replacement reference values. In the context of this function "in between" may also comprise the end values of the described range, i.e. the replacement reference values and the measurement value. In other words, at least in some cases, the corrected value may be the replacement reference value or the actual measurement value. In particular when the estimated error is low or substantially non-existent, the corrected value may be set to be the measurement value. On the other hand, a relatively large error may lead to a correction of the measurement value to a different corrected value. In particular for sets with relatively large errors, each measurement value of the set may be mapped to a corrected value, such that the corrected values are the same or more similar than the original measurement values for the whole set. Since the spectral decomposition is typically determined based on a (relatively small) difference of the measurement values from different spectra, the difference typically is enhanced in the reconstructed image data, e.g. by multiplication with a relatively large factor. Thus, errors in the measurement values can lead to large artifacts due to the errors being larger or much larger than the difference caused by the application of different spectra. Advantageously, the method may thus allow to reduce the effect of these errors. On the other hand, since, the mapping is dependant on the errors, a spectral reconstruction of areas associated with reliable data can still be performed. While some true spectral content of data associated with high errors may be reduced this way, it has been found that, for the end result, it is typically better to avoid an introduction of artifacts than to try to use the spectral information of erroneous measurement values. With large errors any true spectral content would typically be masked by artifacts.

The corrected values are then used as basis for reconstructing computed tomography image data. In particular spectral computed tomography image data may be reconstructed. The method according to the invention may thus provide a relatively simple means of treating unreliable data with less weight during a spectral reconstruction, while still allowing for a spectral reconstruction of areas associated by reliable data.

According to an embodiment, the replacement reference value is defined to be in the order of magnitude of the measurement values of the respective set of measurement values. The order of magnitude may, for example be, defined to be a decimal or binary order of magnitude. Hence, for example, the order of magnitude may correspond to the power of 10 of the respective value. Advantageously, using the same order of magnitude may be a simple means of ensuring that the corrected value, being in between the replacement reference value and the measurement value, will also be of the same order of magnitude as the measurement values. Thus, the corrected value does not deviate too much from the measurement values.

According to an embodiment, the replacement reference value is defined such that it is a linear combination of the measurement values of one set of measurement values. Using a linear combination may provide a relatively simple measure to ensure that the replacement reference value corresponds well to the measurement values of the set.

According to an embodiment, the coefficients of the linear combination are the same within each mapped set. Using the same coefficients may be a particularly simple way of determining the replacement reference values, without the need to determine coefficients for each set individually. Additionally or alternatively, the sum of coefficients of the linear combination is essentially 1. In other words, the linear combination may be a convex combination. A convex combination may allow to achieve a simple means to ensure that the order of magnitude of the in the order of magnitude of the measurement values of the respective set of measurement values.

For example, a corresponding linear combination may be defined as explained in the following. The list $\{S_1, S_2, ... , S_N\}$ may represent attenuation or x-ray intensity measurements from $N$ different spectra for a single detector element readout, in particular detector pixel readout. Hence, $\{S_1, S_2, ... , S_N\}$ are measurement values from a set. For example, in the case of a dual energy CT, the list may be of the form $\{S_1, S_2\}$. A corresponding error estimate may be defined as $w$. The error may be defined in a range of errors, different parts of the range corresponding to different severities of the error. For example, the error estimate may be in the range $[0,1]$, 0 meaning no error and 1 meaning an error so high that the respective set is unsuitable for spectral processing. However, generally, other measures of the error may be applied as well. The replacement reference value $S^*$ may than be defined to be a linear combination of the measurement values from the list of measurement values of the set $\{S_1, S_2, ... , S_N\}$. $S^*$ may in particular be a convex combination of the measurement values $\{S_1, S_2, ... , S_N\}$. Hence, for example, $S^*$ may have the form

$$S^* = \sum_{k=1}^{N} c_k S_k,$$

where

$$c_k \geq 0, k = 1, ..., N \text{ and } \sum_{k=1}^{N} c_k = 1.$$

According to an embodiment, the function that maps the measurement values to corrected values is a monotonic function. Applying a monotonic function may be a particularly useful way of selectively reducing spectral content for sets of measurement values with high errors. Preferably, the function is a smooth monotonic function. For example, measurement values $S_k$ may be mapped by a smooth monotonic function that maps the measurement values $S_k$ to a value between $S_k$ and $S^*$, as defined above. For example, the function may map the measurement values $S_k$ to the corrected values $S_k^{corr}$ in the form of

$$S_k^{\mathrm{corr}} = f(w; S_k),$$

where $f(0; S_k) = S_k$ and $f(1; S_k) = S^*$. Hence, in this example and for an error $w = 1$ the functions may replace the measurement values $S_k$ with the replacement reference value $S^*$, thus setting the corrected values $S_k^{\mathrm{corr}}$ to be the respective replacement reference value $S^*$. On the other hand, in this example, the measurements values $S_k$ are mapped to a value closer to $S^*$ for high errors $w$, and closer to $S_k$ for low errors $w$. Hence, for detector element measurements associated with a high error estimate, the difference between the corrected values $S_k^{\mathrm{corr}}$ of a set is reduced compared to the difference of the measurement values of the set $\{S_1, S_2, \dots, S_N\}$. This reduction, or even complete omission of the difference, leads to a smaller impact of the associated error of the measurement values on the reconstructed image.

According to an embodiment, the replacement reference value is defined to be one of the measurement values of the respective set of measurement values. Using one of the measurement values as replacement reference value may be a particularly simple means of generating the replacement reference value. This embodiment may correspond to a linear combination where one coefficient is 1 and the other coefficients are set to be zero. The replacement reference value may be chosen such that the most reliable of the measurement values is chosen. For example, the most reliable of the measurement values may be chosen based on an estimated error. For example, the measurement value corresponding to a spectrum that in itself has fewer artifacts or provides better statistical properties may be chosen. Alternatively measurement value to be used as replacement reference value may be chosen randomly. This may be beneficial in being particularly simple, while the main advantage of avoiding an increase in error due to calculating a difference between measurement values of the set can still be achieved. For example, in the case of large errors, all the measurement values may be set to take the value of one of the measurement values of the set. Thus, effectively, certain unreliable data, i.e. having a large error estimate, may be treated as non-spectral, since the corrected values all have the same value and thus no spectral difference.

According to an embodiment, the error estimate is based on determining the error of data that is erroneous due to scattered radiation. The error due to scattered radiation may be estimated by a scatter correction algorithm. For example, errors may be caused due to forward scattering or cross scattering reaching the x-ray detector. In a setup with two x-ray sources and detectors at the opposite side of each x-ray source, due to cross scattering, x-rays may be detected by the wrong detector, i.e. the detector that is not opposite the respective x-ray source. Forward scattering is typically known to be present in all x-ray devices. Scattered radiation may lead to additional signals in detector elements.

According to an embodiment, the error estimate is determined based on a ratio, optionally a maximum ratio, between an estimated scatter signal and a total signal strength of the respective measurement values. A maximum error may be understood such, that, for the error estimation, the error of that measurement value of the set is used that yields the highest error estimate. Alternatively other ratios, besides the maximum ratio, may be used. For example, a minimum ratio may be applied. For example, a ratio corresponding to one particular energy and/or one particular spectrum in the set may be applied. For example, two spectra may be acquired. In some cases, it may be advantageous, to reconstruct an image corresponding almost fully to only one of the spectra. In this case, it may be preferable to use the scatter / total ratio corresponding to this spectrum only. A scatter signal can be understood to describe the part of the total signal that is due to scattered x-ray radiation. For example, the scattered x-ray radiation may comprise forward scattering and/or cross scattering. A corresponding error estimate is typically particularly high where the total signal is low and the scattered radiation is high. For example, this is often observed for x-rays passing through both shoulder blades of a patient. Attempting to use affected measurements spectrally may lead to severe streak artifacts along the x-rays passing through both shoulder blades. By applying the method for the reduction of artifacts, the severity of such artifacts can be reduced while leaving areas with lower errors unaffected.

For example, a scattered radiation $S_k^{sc} \geq 0$ may be estimated. The ratio between estimated scatter and total signal may be defined by $S_k^{sc}/S_k$, $k = 1, \dots, N$, $N$ being the number of spectra. This definition may be used to construct an error estimate

$$w = \max_k \min(1, S_k^{sc}/S_k).$$

Hence, a maximum error is determined by choosing the $k$ for which the error is maximal, i.e. hence the error is determined for the measurement value of the set, for which the error is maximal.

Methods for the estimation of scattered radiation are generally known in the state of the art. For example, a scattering may be estimated by model-based profiles and/or by direct measurement of the scattered radiation. An estimation based on model-based profiles may comprise a model of the scatter process which is used to estimate the scattering based on the measured signal. A direct measurement may, for example, be performed by dedicated scatter sensors that are used to measure the scattered radiation. Examples for a model-based estimation and for direct measurement of scattered radiation have been published in the state of the art, such as by Petersilka et al. in "Strategies for scatter correction in dual source CT", Med. Phys. 37 (11), November 2010, 5971-5992.

According to an embodiment, the error estimate is determined based on data errors in a low signal domain, such that a high error is assumed for low detector signals. For example, for low detector signals, statistical errors may render measurements too unreliable to be used for spectral reconstruction. An error estimate may be high for low signals and low for high signals. For example, a particularly big patient may lead to a low signal domain due to a long pathway of the x-rays through the patient's body. For example, some x-ray pathways through an examined object may lead to a complete or nearly complete attenuation of the x-rays in this direction. For example, if the number of photons pear measurement value is lower than a threshold the measurement value may be marked as erroneous. For example, measurement values based on a signal that is lower than a noise level may be determined to be unusable. For example, an error estimate for data errors in a low signal domain may be constructed via the form

$$w = \max_k \cos^2 \left( \frac{\pi}{2} \max \left( 0, min \left( 1, \frac{s_k - s_k^L}{s_k^H - s_k^L} \right) \right) \right),$$

where the parameters $S_k^L$ and $S_k^H$, $k = 1, \dots, N$ define the signal level range where measurements are to be considered as unreliable, i.e. erroneous.

According to an embodiment, the mapping is defined according to at least two different error ranges of the estimate, such that for each error range a different partial mapping function is applied. For example, one error range may correspond to the measurement values being not being changed during the mapping, i.e. corrected values being the same as the measurement values, and a second error range may correspond the measurement values being mapped to be closer or at the corresponding replacement reference value.

According to an embodiment, the error estimate comprises at least three error ranges according to which the mapping is applied, the error ranges comprising a first error range of low errors for which the corrected values are set to be the measurement value, a second error range of medium errors for which the measurement values are mapped such that the corrected values are between the corresponding measurement value and the respective replacement reference value, and a third error range of high errors for which the corrected values are set to be the replacement reference value of their respective set. The first error range may be below a first error threshold. The third error range may be above a second error threshold. The second error range may be between the first and the second error threshold. In particular, the first error range may correspond to errors that are lower than the errors of the second error range of and the second error range may correspond to errors that are lower than the errors of the third error range. The first error range and the third error range may each be directly adjacent to the second error range. Optionally, there may be more than three error ranges. For example, there may be different types of medium error ranges, such that a different mapping is applied for the different types of medium error ranges.

The embodiments described herein may be combined with each other unless indicated otherwise. For example, the replacement reference values may be defined via a linear combination and, at the same time, the mapping may be defined via error ranges.

According to a further aspect of the invention, a method for providing spectral computed tomography image data is provided. The method comprises

-    acquiring measurement data by performing a spectral computed tomography scan,
-    performing the method for the reduction of artifacts in spectral computed tomography image data as described herein.

All features and advantages of the method for the reduction of artifacts may be adapted to the method for providing spectral computed tomography image data and vice versa.

According to a further aspect of the invention, a computer program comprising instructions which, when the program is executed by a computer, cause the computer to carry out the method as described herein, in particular the method for the reduction of artifacts in spectral computed tomography image data. All features and advantages of the method for the reduction of artifacts and of the method for providing spectral computed tomography image data may be adapted to the computer program and vice versa.

According to a further aspect of the invention, a computer-readable storage medium, in particular non-transient storage medium, comprising instructions which, when executed by a computer, cause the computer to carry out the method as described herein, in particular the method for the reduction of artifacts in spectral computed tomography image data. All features and advantages of the method for the reduction of artifacts, of the method for providing spectral computed tomography image data, and of the computer program may be adapted to the computer-readable storage medium and vice versa. The storage medium may be any computer-readable storage medium. For example, the storage medium may be an optical storage medium or a solid-state storage medium. The storage medium may be a hard-drive, a solid-state-disk a flash storage, an online server etc.

According to a further aspect of the invention, a computed tomography system comprising a processing unit that is configured to carry out any of the methods as described herein, is provided. All features and advantages of the method for the reduction of artifacts, of the method for providing spectral computed tomography image data, of the computer program, and of the computer-readable storage medium may be adapted to the computed tomography system and vice versa.

The accompanying drawings illustrate various exemplary embodiments and methods of various aspects of the invention.

Fig. 1 shows a flow diagram of a method for the reduction of artifacts in spectral computed tomography image data according to an embodiment of the invention;

Fig. 2 shows a mapping function according to an embodiment of the invention;

Fig. 3 shows a reconstructed spectral computed tomography image, where measurement values have not been corrected according to the invention;

Fig. 4 shows a reconstructed spectral computed tomography image based on corrected values according to the invention;

Fig. 5 shows a flow diagram of a method for providing spectral computed tomography image data according to an embodiment of the invention; and

Fig. 6 a computed tomography system according to an embodiment of the invention.

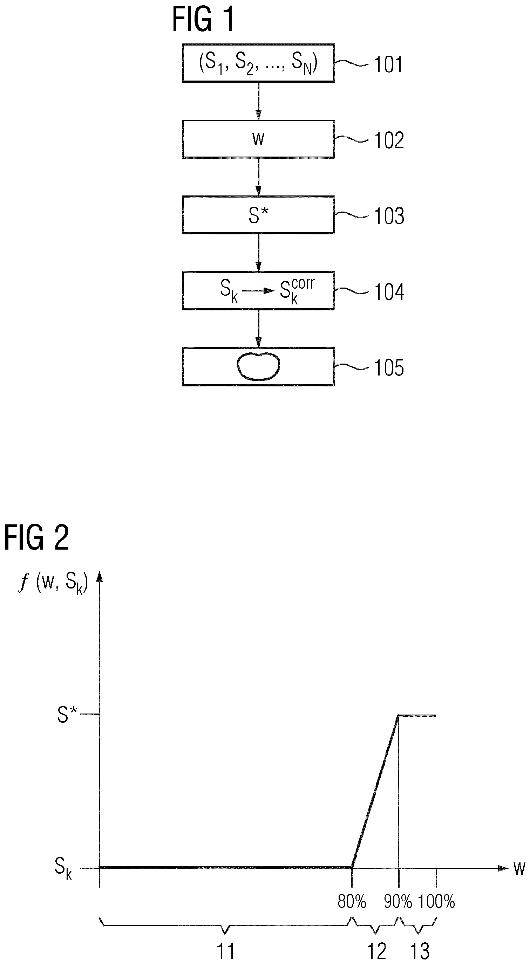

Figure 1 shows a flow diagram of a method for the reduction of artifacts in spectral computed tomography image data according to an embodiment of the invention. In a first step 101 computed tomography measurement data with sets of measurement values are received. One set $\{S_1, S_2, ... , S_N\}$ corresponds to one detector element readout of the measurement data and comprises measurement values $S_k$ ($k = 1, 2, ... N$) from $N$ different x-ray spectra. $N$ is at least two, hence, there are at least 2 two different x-ray spectra. In the case of exactly two different x-ray spectra per set, the set has the form $\{S_1, S_2\}$. This particular example of two different x-ray spectra per set may correspond to dual energy CT.

In a further step 102, an error estimate $w$ is determined and/or received per set of measurement values. For example, the error estimate may be based on determining the error of data that is erroneous due to scattered radiation. An error estimate for scattered radiation may be determined, for example, via a maximum ratio between an estimated scatter signal $S_k^{sc} \geq 0$ and a total signal strength $S_k$ of the respective measurement values. For example, such an error $w$ may be determined according to the term

$$w = \max_k \min(1, S_k^{sc}/S_k).$$

Additionally or alternatively, the error estimate $w$ may be determined based on data errors in a low signal domain, such that a high error is assumed for low detector signals, such as based on parameters $S_k^L$ and $S_k^H$, $k = 1, ...,N$ defining the signal level range where measurements are to be considered as unreliable, e.g.:

$$w = \max_k \cos^2\left(\frac{\pi}{2}\max\left(0, min\left(1, \frac{S_k - S_k^L}{S_k^H - S_k^L}\right)\right)\right).$$

In a further step 103, for at least some of the sets, a replacement reference value $S^*$ per set of measurement values is defined. Optionally, the replacement reference value may be defined such that it is a linear combination of the measurement values of one set of measurement values, e.g. as a convex combination

$$S^* = \sum_{k=1}^{N} c_k S_k.$$

Preferably, the sum of coefficients $c_k$ of the linear combination is essentially 1, i.e., $\sum_{k=1}^{N} c_k = 1$ . In on example, one of the coefficients $c_k$ is 1 and the other coefficients are zero. Hence, in this example, the replacement reference value is defined to be one of the measurement values of the respective set of measurement values.

In a further step 104, a function that maps the measurement values $S_k$ to corrected values $S_k^{\mathrm{corr}}$ is applied. The corrected values $S_k^{\mathrm{corr}}$ are in between the mapped measurement value $\boldsymbol{S_k}$ and the replacement reference value $\boldsymbol{S^*}$ of the respective set. The distance of the corrected value to each of $\boldsymbol{S^*}$ and $\boldsymbol{S_k}$ depends on the error estimate w such that for larger errors the corrected value is closer to or at the replacement reference value $\boldsymbol{S^*}$ and for smaller errors the corrected value $S_k^{\mathrm{corr}}$ is closer to or at the measurement value $\boldsymbol{S_k}$. For example, a function $\boldsymbol{f(w;S_k)}$, in particular monotonic function, may be used for mapping the measurement values $\boldsymbol{S_k}$ to the corrected values $S_k^{\mathrm{corr}} = f(w; S_k)$ . For example, assuming an error estimate ranging from 0 for no error to 1 for unsuitably large error, the function may yield $\boldsymbol{f(0;S_k)} = \boldsymbol{S_k}$ and $\boldsymbol{f(1;S_k)} = \boldsymbol{S^*}$. Optionally, the mapping may be defined according to multiple error ranges of the error estimate, such that for different error ranges a different partial mapping function is applied.

One example of a mapping function having different error ranges 11, 12, 13 is shown in figure 2. In the embodiment shown in figure 2, the error estimate ranges from 0% estimated error to 100% estimated error, where 100% error means the corresponding set of measurement values is completely unsuitable for spectral decomposition. While, in this representation, the replacement reference value is above the measurement value, it can in principle be higher or lower than the measurement value. The function $\boldsymbol{f(w;S_k)}$ is separated into three ranges 11-13, a first range 11 below 80%, a second range 12 between 80% and 90%, and a third 13 range above 90%. In the first range 11, i.e. below an error estimate of 80%, the function maps the measurement value such that the corrected value is the measurement value $S_k^{\mathrm{corr}} = f(w; S_k) = S_k$ . Hence, the measurement value is not changed in this first range 11. In the second range 12, i.e. between an error estimate of 80% and 90%, the function maps the measurement value such that the corrected value $S_k^{\mathrm{corr}}$ is between the measurement value $\boldsymbol{S_k}$ and the replacement reference value $\boldsymbol{S^*}$. Accordingly, in the second range 12, the measurement value is corrected to some degree. In the third range 13, i.e. above an error estimate of 90%, the function maps the measurement value such that the corrected value is the replacement reference value $S_k^{\mathrm{corr}} = f(w; S_k) = S^*$ . Accordingly, in the third range 13, measurement values are corrected maximally.

Referring back to figure 1, in a further step 105, computed tomography image data is reconstructed based on the corrected values.

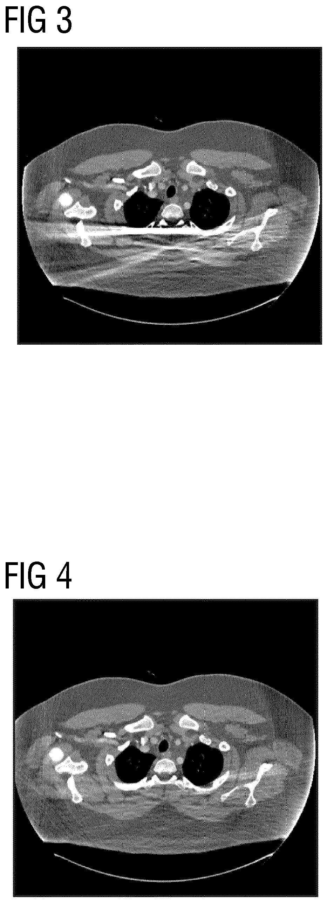

Figure 3 shows a reconstructed spectral computed tomography image, where measurement values have not been corrected according to the invention. On the other hand, figure 4, shows a spectral computed tomography image, where corrected values that have been corrected according to the invention have been used for the image reconstruction. In Figure 3, depicting an axial CT image from a dual source thorax examination, one can clearly see several (white) streak artifacts passing about horizontally through one scapula, ribs, and vertebra. In fig-ure 4, these artifacts have mostly been eliminated due to the application of a method according to the invention.

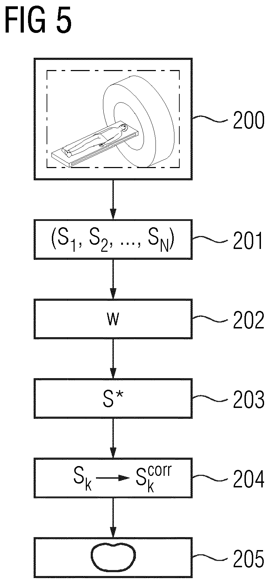

Figure 5 shows a flow diagram of a method for providing spectral computed tomography image data according to an embodiment of the invention. In a first step 200, measurement data are acquired by performing a spectral computed tomography scan. The following steps 201-205 may be applied equivalently to the steps 101-105 as described with respect to figure 1.

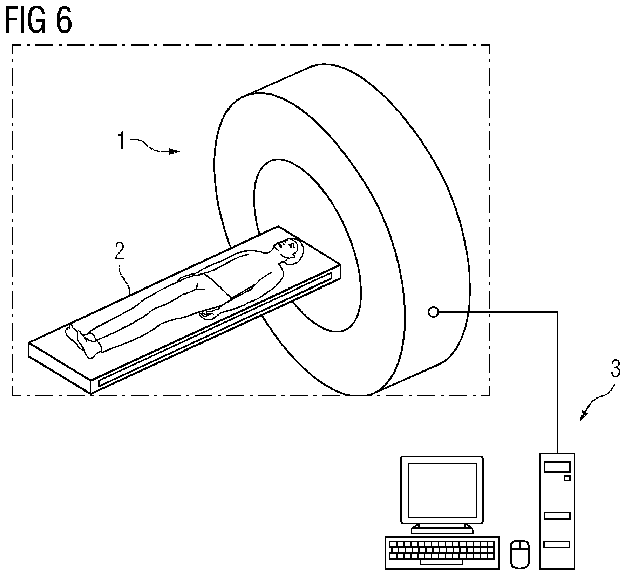

Figure 6 shows a computed tomography system according to an embodiment of the invention. In this embodiment, the computed tomography system comprises a gantry 1, a movable patient table 2 and a processing unit 3 that is configured to carry out a method as described herein, for example, as described with respect to figure 5.

**Claims**

**1.** A computer-implemented method for the reduction of artifacts in spectral computed tomography image data, the method comprising:

(a) receiving computed tomography measurement data with sets of measurement values, wherein each set corresponds to a respective detector element readout and comprises measurement values for at least two different x-ray spectra;
(b) determining and/or receiving one error estimate per set of measurement values;
(c) defining one replacement reference value per set of measurement values;
(d) applying a function that maps the measurement values to corrected values, wherein for each measurement value a respective corrected value is in between that measurement value and the replacement reference value for the set comprising that measurement value, the distance of the respective corrected value to each depending on the error estimate such that for larger errors the respective corrected value is closer to or at the replacement reference value for the set comprising that measurement value and for smaller errors the respective corrected value is closer to or at that measurement value;
(e) reconstructing computed tomography image data based on the corrected values.

2. The method according to claim 1,
wherein the replacement reference value for the set comprising that measurement value is defined to be in the order of magnitude of the measurement values of the set comprising that measurement value.

3. The method according to any one of the preceding claims, wherein the replacement reference value for the set comprising that measurement value is defined such that it is a linear combination of the measurement values of the set comprising that measurement value.

4. The method according to claim 3,

wherein coefficients of the linear combination are the same within each mapped set and/or
wherein the sum of coefficients of the linear combination is essentially 1.

5. The method according to any one of the preceding claims, wherein the replacement reference value for the set comprising that measurement value is defined to be one of the measurement values of the set comprising that measurement value.

6. The method according to any one of the preceding claims, wherein the error estimate is based on determining the error of data that is erroneous due to scattered radiation.

7. The method according to claim 6,
wherein the error estimate is determined based on a ratio, optionally a maximum ratio, between an estimated scatter signal and a total signal strength of the measurement values of the respective set.

8. The method according to any one of the preceding claims, wherein the error estimate is determined based on data errors in a low signal domain, such that a high error is assumed for low detector signals.

9. The method according to any one of the preceding claims, wherein the function that maps the measurement values to corrected values is a monotonic function.

10. The method according to any one of the preceding claims, wherein the mapping is defined according to at least two different error ranges of the estimate, such that for each error range a different partial mapping function is applied.

11. The method according to any one of the preceding claims, wherein the error estimate comprises at least three error ranges (11, 12, 13) according to which the mapping is applied, the error ranges (11, 12, 13) comprising a first error range (11) of low errors for which the respective corrected value is set to be that measurement value, a second error range (12) of medium errors for which the measurement values are mapped such that the respective corrected value is between that measurement value and the replacement reference value for the set comprising that measurement value, and a third error range (13) of high errors for which the respective corrected value is set to be the replacement reference value for the set comprising that measurement value.

12. A method for providing spectral computed tomography image data, the method comprising:

- acquiring measurement data by performing a spectral computed tomography scan,
- performing the method according to any one of the preceding claims.

13. A computer program comprising instructions which, when the program is executed by a computer, cause the computer to carry out the method of any one of claims 1 to 12.

14. A computer-readable storage medium, in particular non-transient storage medium, comprising instructions which, when executed by a computer, cause the computer to carry out the method of any one of claims 1 to 12.

15. A computed tomography system comprising a processing unit (3) that is configured to carry out the method according to any one of claims 1 to 12.

# FIG 1

$(S_1, S_2, ..., S_N)$ — 101

↓

w — 102

↓

$S*$ — 103

↓

$S_k \longrightarrow S_k^{corr}$ — 104

↓

105

# FIG 2

$f(w, S_k)$

$S*$

$S_k$

80%  90% 100%  w

11  12  13

# FIG 3

# FIG 4

# FIG 5

200

$(S_1, S_2, ..., S_N)$ — 201

$W$ — 202

$S^*$ — 203

$S_k \longrightarrow S_k^{corr}$ — 204

205

FIG 6

Europäisches
Patentamt

European
Patent Office

Office européen
des brevets

# EUROPEAN SEARCH REPORT

**Application Number**

EP 24 15 6011

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ERATH JULIEN ET AL: "Deep learning-based forward and cross-scatter correction in dual-source CT", MEDICAL PHYSICS., vol. 48, no. 9, 26 July 2021 (2021-07-26), pages 4824-4842, XP093183871, US ISSN: 0094-2405, DOI: 10.1002/mp.15093 | 1,2, 5-10, 12-15 | INV. G06T11/00 |
| A | * the whole document * | 3,4,11 | |
| X | Erath, Julien Frank Josef: "Korrektur von Streustrahlartefakten durch tiefe neuronale Netze in der klinischen Computertomographie", Dissertation, Ruprecht-Karls-Universität, 30 January 2023 (2023-01-30), XP093183831, Heidelberg Retrieved from the Internet: URL:http://www.ub.uni-heidelberg.de/archiv /32391 [retrieved on 2024-07-09] | 1,13-15 | |
| A | * sec. 3.1.4, "Streustrahlsubtraktion" * * sec. 3.2.6, "Korrektur der Längs- und Querstreuung im Dual-Energy CT" * | 2-12 | TECHNICAL FIELDS SEARCHED (IPC) G06T |

The present search report has been drawn up for all claims

| Place of search | Date of completion of the search | Examiner |
|---|---|---|
| Munich | 10 July 2024 | Werling, Alexander |

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PETERSILKA et al.** Strategies for scatter correction in dual source CT. *Med. Phys.*, November 2010, vol. 37 (11), 5971-5992